# EUROPÄISCHE PATENTSCHRIFT

(11) **EP 3 713 513 B1**
(45) Veröffentlichungstag und Bekanntmachung des Hinweises auf die Patenterteilung: **02.03.2022**
(21) Anmeldenummer: 18826188.7
(22) Anmeldetag: 23.11.2018
(51) Int. Cl.: A61C 13/00

(54) **VERFAHREN ZUR HERSTELLUNG EINES ZAHNERSATZTEILS**
METHOD FOR PRODUCING A DENTAL PROSTHESIS PART
PROCÉDÉ DE FABRICATION D'UNE PROTHÈSE DENTAIRE

(30) Priorität: 23.11.2017 DE 102017221002
(43) Veröffentlichungstag der Anmeldung: 30.09.2020
(73) Patentinhaber: Sirona Dental Systems GmbH, 64625 Bensheim (DE)
(72) Erfinder: RABE, Susanne, 64297 Darmstadt (DE)
(74) Vertreter: Özer, Alpdeniz
(86) Internationale Anmeldenummer: PCT/EP2018/082446
(87) Internationale Veröffentlichungsnummer: WO 2019/101967

(56) Entgegenhaltungen:
- EP-A1- 2 450 000
- WO-A2-2009/090214
- WO-A2-2011/017113
- DE-A1- 19 950 284
- GB-A- 2 148 872
- US-A1- 2007 050 074

## Beschreibung

### Technisches Gebiet

Die Erfindung betrifft ein Verfahren zur Herstellung eines Zahnersatzteils sowie ein Zahnersatzteil.

### Stand der Technik

Größere permanente Zahnersatzteile, wie permanente Brückenversorgungen werden typischerweise aus besonders stabilen Werkstoffen, wie Zirkonoxid oder einem Metall, hergestellt. Sogenannte dentale Komposite sind einfacher zu verarbeiten und günstiger, jedoch aufgrund ihrer physikalischen Eigenschaften für die Herstellung von permanenten Brückenversorgungen nicht geeignet.

Aus der US 2007/0050074 A1 ist die automatische Herstellung eines Zahnersatzteils durch eine Fräsmaschine oder durch Laser-Lithorahie unter Berücksichtigung von Datensätzen von einer Belastungsuntersuchung von Zahnersatz mittels Finite-Elemente-Verfahren bekannt.

Aus der GB 2 148 872 A ist bekannt, eine vorgefertigte Verstärkungseinlage in einer gegossenen Krone oder in einer zunächst aus einem Pulver gepressten und dann gesinterten Krone vorzusehen.

Um die Vorzüge von Komposit-Materialien dennoch nutzen zu können, ist es aus dem Artikel "Clinical and laboratory procedures to fabricate fibre reinforced composite fixed partial denture", G. Rappelli et al., cosmetic dentistry no. 4, 2008, bekannt, eine dentale Brückenkonstruktion aus einem Komposit-Material und einer verstärkenden Glasfaser herzustellen, wobei eine Gussform mit dem Komposit-Material und Schichten von Glasfasern gefüllt und das Komposit-Material durch Photopolymerisation gehärtet wird.

Ferner existieren faserverstärkte Komposite, sogenannte fiber reinforced composites, für zahnmedizinische Anwendungen, z.B. everX Posterior der Fa. GC Corporation, Tokyo, Japan. Das Material setzt sich im Wesentlichen aus einer Polymermatrix und kurzen Glasfasern als Füller zusammen und eignet sich gemäß Herstellerangaben zur Versorgung von größeren Kavitäten. Das Material wird, wie für dentale Komposite üblich, in Schichten in die Kavität eingebracht und mittels Polymerisation gehärtet wird.

Vor diesem Hintergrund besteht die Aufgabe der vorliegenden Erfindung darin, den Stand der Technik weiterzuentwickeln und ein möglichst zuverlässiges und kostengünstiges Herstellungsverfahren für ein Zahnersatzteil sowie ein stabiles und kostengünstiges Zahnersatzteil bereitzustellen.

Darstellung der Erfindung Ein Gegenstand der Erfindung ist ein Herstellungsverfahren für ein Zahnersatzteil gemäß Anspruch 1.

Bevorzugte Ausführungsformen sind in den abhängigen Ansprüchen enthalten.

Das erfindungsgemäße Verfahren ist auf das Herstellen eines Zahnersatzteils mittels eines 3D-Druckers gerichtet, wobei ein dreidimensionales digitales Zahnersatzteilmodell unter Berücksichtigung eines digitalen Gebissmodells computergestützt erzeugt wird, das digitale Gebissmodell einen zu versorgenden bezahnten und/oder unbezahnten Bereich eines Unterkiefers und/oder Oberkiefers umfasst und unter Berücksichtigung des Gebissmodells computergestützt eine Belastungsuntersuchung des Zahnersatzteilmodells durchgeführt wird.

Unter Berücksichtigung eines Ergebnisses der Belastungsuntersuchung wird ein dreidimensionales digitales erstes Verstärkungsstrukturmodell mit einer ersten dreidimensionalen Form und einer ersten Position computergestützt innerhalb des Zahnersatzteilmodells angeordnet, das Zahnersatzteilmodell um ein dem angeordneten ersten Verstärkungsstrukturmodell entsprechendes Volumen reduziert und das Zahnersatzteilmodell in ein unteres Teilvolumen und ein oberes Teilvolumen aufgeteilt, wobei das untere Teilvolumen und das obere Teilvolumen jeweils an das angeordnete erste Verstärkungsstrukturmodell angrenzen.

Ein unterer Teil des Zahnersatzteils wird gemäß dem unteren Teilvolumen des Zahnersatzteilmodells mittels eines 3D-Druckers aus einem Komposit-Material erzeugt, eine dem ersten Verstärkungsstrukturmodell entsprechende erste Verstärkungsstruktur auf dem unteren Teil des Zahnersatzteils angeordnet und ein oberer Teil des Zahnersatzteils gemäß dem oberen Teilvolumen des Zahnersatzteilmodells mittels des 3D-Druckers aus dem Komposit-Material auf den unteren Teil des Zahnersatzteils mit der angeordneten ersten Verstärkungsstruktur gesetzt, um das Zahnersatzteil fertig zu stellen.

Gemäß einer Weiterbildung wird mindestens ein weiteres Verstärkungsstrukturmodell mit einer weiteren Form und einer weiteren Position oberhalb und/oder neben dem ersten Verstärkungsstrukturmodell innerhalb des Zahnersatzteilmodells computergestützt angeordnet, das Zahnersatzteilmodell um dem angeordneten erste Verstärkungsstrukturmodell und dem mindestens einen weiteren Verstärkungsstrukturmodell entsprechende Volumina reduziert und das reduzierte Zahnersatzteilmodell in ein unteres Teilvolumen und ein oberes Teilvolumen und für jedes oberhalb der ersten Verstärkungsstruktur positionierte weitere Verstärkungsstrukturmodell in ein weiteres mittleres Teilvolumen aufgeteilt. Schrittweise wird jeweils ein Teil des Zahnersatzteils gemäß einem der Teilvolumina mittels eines 3D-Druckers aus einem Komposit-Material erzeugt und eine einem der Verstärkungsstrukturmodelle entsprechende Verstärkungsstruktur an der jeweiligen Position angeordnet.

Das Gebissmodell wird beispielsweise aus zweidimensionalen oder dreidimensionalen Aufnahmen z.B. mittels einer intraoralen Kamera erzeugt werden. Es versteht sich, dass das Gebissmodell die für die Versorgung notwendigen Informationen zu dem zu versorgenden Bereich, z.B. die Konturen angrenzender Zähne, Präparationsstellen, Implantate mit Aufbauten umfasst. Das Zahnersatzteilmodell wird manuell mittels eines geeigneten Eingabemittels oder automatisch erzeugt. Beispielsweise wird ein Modellzahn oder werden mehrere Modellzähne aus einer Zahndatenbank virtuell an einer bzw. mehreren Fehlstellen in dem Gebissmodell positioniert, gegebenenfalls größenmäßig angepasst und um Strukturen erweitert, welche den Präparationsstellen bzw. präparierten Bereichen an benachbarten Zähne entsprechen.

Die Belastungsuntersuchung wird gemäß einer ersten Ausführungsform mittels Finite-Elemente-Methode durchgeführt. Es versteht sich, dass das Gebissmodell für die Belastungsuntersuchung vorteilhafterweise zu dem zu versorgenden Bereich benachbarte Zähne und/oder gegenüberliegende Zähne umfasst. In einer Weiterentwicklung ist auch das Kiefergelenk bzw. eine Kiefergelenks- bzw. Kaubewegung vom Gebissmodell umfasst und wird der Belastungsuntersuchung mit zugrunde gelegt. So können im Rahmen der Belastungsuntersuchung nicht nur die Geometrie des Zahnersatzteils selbst, sondern auch die Einflüsse der späteren Umgebung mit berücksichtigt werden.

Anhand des Ergebnisses der Belastungsuntersuchung wird automatisch direkt durch den Rechner und/oder manuell durch einen Anwender eine Verstärkungsstruktur bzw. ein virtuelles Modell der Verstärkungsstruktur oder gemäß der Weiterbildung auch zwei oder mehr Verstärkungsstrukturmodelle innerhalb des Zahnersatzteils positioniert.

Als Verstärkungsstrukturmodell wird gemäß einer ersten Ausführungsform ein dreidimensionales digitales Faserbündelmodell angeordnet, wobei die Form des Faserbündelmodells eine Länge und einen Durchmesser aufweist. Zwischen dem Drucken des unteren und des oberen Teils des Zahnersatzteils wird ein dem Faserbündelmodell in Länge und Durchmesser entsprechendes Faserbündel auf dem unteren Teil des Zahnersatzteils angeordnet.

Alternativ wird die Verstärkungsstruktur aus einem kurzfaserverstärkten Komposit-Material ausgebildet und mittels des 3D-Druckers erzeugt. Hierzu wird das Verstärkungsstrukturmodell mit einer Form und Position digital geplant und zwischen dem Erzeugen des unteren und des oberen Teils des Zahnersatzteils eine dem Modell entsprechende Verstärkungsstruktur aus dem kurzfaserverstärkten Komposit-Material gedruckt. Der 3D-Drucker weist hierfür beispielsweise eine weitere Düse auf. Als erstes Komposit-Material wird ein übliches dentales Komposit verwendet. Typische Komposit-Materialien bestehen aus einer organischen Kunststoffmatrix, die mit anorganischen Füllkörpern versetzt ist. In einer bevorzugten Ausführungsform beträgt ein Massenanteil der organischen Kunststoffmatrix des ersten Komposit-Materials höchstens 40% und ein Massenanteil der anorganischen Füllkörper mindestens 60%.

Das erste Komposit-Material wird mittels des 3D-Druckers beispielsweise schichtweise verfestigt, um die jeweiligen Teile des Zahnersatzteils zu erzeugen. Gemäß einer bevorzugten Weiterbildung geschieht das Verfestigen mittels Photopolymerisation, also mittels Lichteintrags mit einer geeigneten Strahlungsquelle.

Mittels der mindestens einen Verstärkungsstruktur, umfassend ein Faserbündel oder ein kurzfaserverstärktes Komposit, erhält das Zahnersatzteil, welches ansonsten aus einem üblichen Komposit-Material besteht eine hohe Stabilität. Mit dem erfindungsgemäßen Verfahren können daher auch größere Brückenversorgungen und/oder dauerhafter Zahnersatz hergestellt werden.

Ein Vorteil des erfindungsgemäßen Verfahrens ist, dass die Herstellung durch den Einsatz des 3D-Druckers besonders einfach, kostengünstig und schnell ist. Durch die Aufteilung des Zahnersatzteilmodells ist eine einfache, schrittweise und schnelle Herstellung mittels 3D-Drucker möglich.

Es versteht sich, dass eine Positionierung einer als Faserbündel ausgebildeten Verstärkungsstruktur auf dem zuerst hergestellten Teil des Zahnersatzteils besonders zuverlässig ist, wenn das erste Teilvolumen bereits teilweise um den die Verstärkungsstruktur aufnehmenden Volumenbereich herum reicht. Gemäß einer Weiterbildung weist das untere Teilvolumen und/oder jedes mittlere Teilvolumen zumindest eine Vertiefung zur Aufnahme der ersten bzw. einer weiteren Verstärkungsstruktur auf.

Weiterhin versteht es sich, dass im Falle eines Faserbündels als Verstärkungsstruktur das erste Teilvolumen noch nicht zu weit um den die Verstärkungsstruktur aufnehmenden Volumenbereich herumreichen sollte, um Beschädigungen des bereits hergestellten unteren Teils des Zahnersatzteils beim Positionieren des Faserbündels zu vermeiden. Ist die Verstärkungsstruktur als Faserbündel ausgebildet, so ist es entsprechend vorteilhaft, wenn das erste Teilvolumen ohne Hinterschnitte ausgebildet wird. Ist die mindestens eine Verstärkungsstruktur als Faserbündel ausgebildet, so ist es besonders bevorzugt, das um die mindestens eine Verstärkungsstruktur reduzierte Zahnersatzteilmodell so in Teilvolumina aufzuteilen, dass alle Teilvolumina in an eine der Verstärkungsstrukturen angrenzenden Bereichen keine Hinterschnitte aufweisen.

Ein weiterer Vorteil des erfindungsgemäßen Herstellungsverfahrens ist, dass es sehr zuverlässig, schnell und einfach durchführbar ist und zu einem sehr stabilen und kostengünstigen Zahnersatzteil führt.

Vorteilhafterweise wird das Gebissmodell anhand von mehreren zweidimensionalen optischen Aufnahmen berechnet. Eine einfache Möglichkeit eine Zahnsituation eines Patienten zu erfassen ist das Vermessen mittels intraoraler Kamera, wobei die Kamera beispielsweise mehrere zweidimensionale Aufnahmen erzeugt. Aus den Aufnahmen kann dann auf bekannte Weise ein dreidimensionales Modell der vermessenen Zahnsituation, also ein Gebissmodell erzeugt werden.

Vorteilhafterweise ist das Komposit-Material biokompatibel. Hierdurch ist das Komposit-Material zur Verwendung für eine dentale Versorgung geeignet.

Vorteilhafterweise wird das Zahnersatzteil mittels eines Photopolymerisationsverfahrens gedruckt und das Komposit-Material enthält Initiatoren und Stabilisatoren für das Photopolymerisationsverfahren.

Vorteilhafterweise umfasst die als Faserbündel ausgebildete mindestens eine Verstärkungsstruktur Glasfasern oder Kohlefasern oder Keramikfasern oder Fasern aus Aluminiumoxid oder eine Kombination unterschiedlicher Fasern. Alternativ oder als Weiterbildung umfasst das mindestens eine Faserbündel mehrere in einer Matrix aus biokompatiblem Material eingebettete Fasern, wobei die Matrix gemäß einer Weiterbildung aus einem organischen Harz besteht und/oder Initiatoren und Stabilisatoren für ein Photopolymerisationsverfahren beinhaltet.

Vorteilhafterweise wird die Dicke der mindestens einen als Faserbündelmodell ausgebildeten Verstärkungsstruktur aus einer vorgegebenen Menge von Dicken ausgewählt, wodurch sichergestellt wird, dass ein dem Faserbündelmodell entsprechendes Faserbündel vorhanden ist. Faserbündel liegen typischerweise in bestimmten Stärken bzw. Dicken vor, die nicht beliebig angepasst werden können. Die Länge hingegen kann beliebig gekürzt und somit an das Faserbündelmodell angepasst werden. Durch die Matrix entsteht ein sogenannter Faserverbundwerkstoff. Die Kombinationsmöglichkeiten aus Fasermaterial und Matrixmaterial ergeben viele Freiheitsgrade, um die verstärkende Struktur, hier als Faserbündel bezeichnet, zu gestalten.

Kurzbeschreibung der Zeichnungen Ausführungsbeispiele der Erfindung sind in der Zeichnung dargestellt. Es zeigt die
- Fig. 1: einen schematischen Ablauf einer ersten Ausführungsform des erfindungsgemäßen Herstellungsverfahrens,
- Fig. 2: eine schematische Ansicht einer ersten Ausführungsform eines das erfindungsgemäße Verfahren hergestellten Zahnersatzteils,
- Fig. 3: eine schematische Ansicht einer zweiten Ausführungsform eines durch das erfindungsgemäße Verfahren hergestellten Zahnersatzteils.

### Ausführungsbeispiele

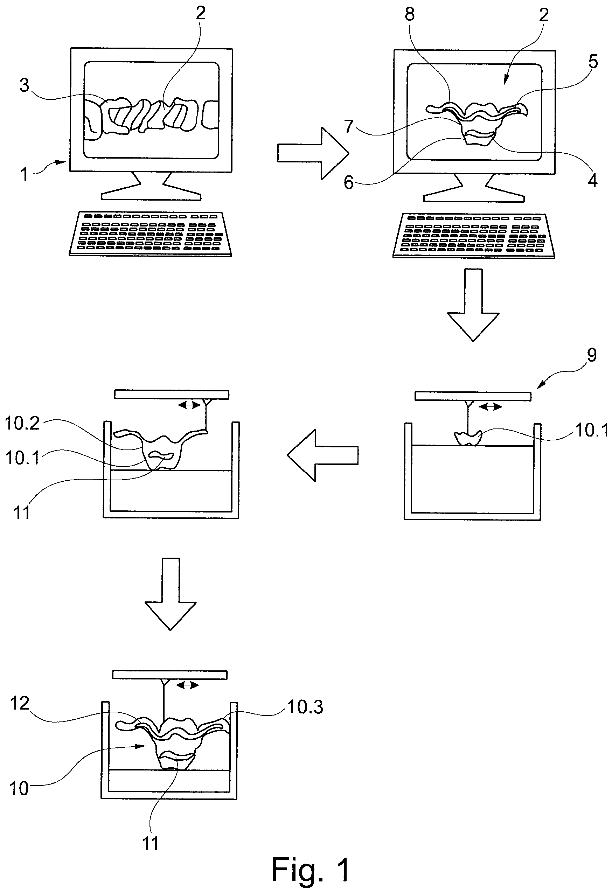

Die Fig. 1 zeigt sechs Verfahrensschritte gemäß einer ersten erfindungsgemäßen Ausführungsform eines Herstellungsverfahrens für ein Zahnersatzteil 10.

Mittels einer Recheneinheit 1 wird ein dreidimensionales digitales Zahnersatzteilmodell 2 erzeugt und eine Belastungsuntersuchung durchgeführt. Für das Entwerfen bzw. Erzeugen des Zahnersatzteilmodells 2 sowie für die Belastungsuntersuchung des Zahnersatzteilmodells 2 wird ein digitales Gebissmodell 3 herangezogen, welches einen zu versorgenden bezahnten und/oder unbezahnten Bereich eines Unterkiefers und/oder Oberkiefers umfasst. Das Zahnersatzteilmodell 2 wird in das Gebissmodell 3 hinein geplant und/oder an das Gebissmodell 3 angepasst. Gemäß einer ersten Ausführungsform wird das Zahnersatzteilmodell 2 für die Belastungsuntersuchung in dem Gebissmodell positioniert und unter anderem eine Kaubewegung simuliert.

Anhand des Ergebnisses der Belastungsuntersuchung werden im dargestellten Ausführungsbeispiel ein oberes und ein unteres dreidimensionales digitales Verstärkungsstrukturmodell 4, 5 computergestützt innerhalb des Zahnersatzteilmodells 3 angeordnet. Die Anzahl, die jeweilige dreidimensionale Form sowie die jeweilige Position der Verstärkungsstrukturmodelle 4, 5 wird vollautomatisch durch die Recheneinheit 1 bestimmt oder rein manuell durch einen Anwender mittels geeigneter Eingabemittel oder durch eine ein Kombination aus automatischen Schritten, z.B. Vorschläge für Position und Form und manuellen Schritten, z.B. optionale Änderung der Lage oder Form. Das Zahnersatzteilmodell 2 wird anschließend um ein den angeordneten Verstärkungsstrukturmodellen 4, 5 entsprechendes Volumen, also um die von den Verstärkungsstrukturmodellen 4, 5 eingenommenen Volumenbereiche reduziert und in ein unteres Teilvolumen 6, ein mittleres Teilvolumen 7 und ein oberes Teilvolumen 8 aufgeteilt, wobei das untere Teilvolumen 6 von einer unteren Oberfläche des Teilvolumens 6 bis zu dem unteren Verstärkungsstrukturmodell 4 reicht, das mittlere Teilvolumen 7 sich von dem unteren Verstärkungsstrukturmodell 4 zu dem oberen Verstärkungsstrukturmodell 5 erstreckt und das obere Teilvolumen 8 von dem oberen Verstärkungsstrukturmodell 5 bis zu einer der ersten Oberfläche gegenüberliegenden zweiten Oberfläche des Zahnersatzteilmodells 2 reicht. Das Zahnersatzmodell 2 wird jeweils entlang des Verlaufs der Verstärkungsstrukturmodelle 4, 5 aufgeteilt.

Anschließend wird das Zahnersatzteil 10 in mehreren Schritten hergestellt. Zunächst wird ein unterer Teil 10.1 des Zahnersatzteils 10 gemäß dem unteren Teilvolumen 6 des Zahnersatzteilmodells 2 mittels eines 3D-Druckers 9 aus einem Komposit-Material erzeugt. Anschließend wird das untere Verstärkungsstrukturmodell 4 angeordnet.

Ist das Verstärkungsstrukturmodell 4 einer ersten Ausführungsform entsprechend als Faserbündelmodell geplant bzw. ausgelegt, so wird ein dem Verstärkungsstrukturmodell 4 in der Form, also insbesondere hinsichtlich einer Länge und einer Dicke entsprechendes erstes Faserbündel als untere Verstärkungsstruktur 11 auf dem unteren Teil 10.1 des Zahnersatzteils 10 an der für das untere Verstärkungsstrukturmodell 4 geplanten Position angeordnet.

Soll die Verstärkungsstruktur 11 einer zweiten Ausführungsform entsprechend aus einem kurzfaserverstärkten Komposit-Material bestehen, so wird ein dem Verstärkungsstrukturmodell 4 hinsichtlich der dreidimensionalen Form entsprechender Körper aus dem kurzfaserverstärkten Komposit-Material als untere Verstärkungsstruktur 11 an der für das untere Verstärkungsstrukturmodell 4 geplanten Position angeordnet. Eine solche Verstärkungsstruktur 11 wird gemäß einer ersten Alternative vorab oder parallel erzeugt, z.B. mittels eines weiteren 3D-Druckers und anschließend auf dem unteren Teil 10.1 des Zahnersatzteils 10 angeordnet. Alternativ wird die Verstärkungsstruktur 11 mittels desselben 3D-Druckers direkt auf dem unteren Teil 10.1 des Zahnersatzteils 10 erzeugt.

Dann wird ein mittlerer Teil 10.2 des Zahnersatzteils 10 gemäß dem mittleren Teilvolumen 7 mit dem 3D-Drucker 9 aus dem Komposit-Material auf den unteren Teil 10.1 des Zahnersatzteils 10 und die untere Verstärkungsstruktur 11 gesetzt.

Eine dem oberen Verstärkungsstrukturmodell 5 entsprechende obere Verstärkungsstruktur 12 wird an der für das obere Verstärkungsstrukturmodell 5 geplanten Position auf dem mittleren Teil 10.2 des Zahnersatzteils 10 angeordnet. Bevorzugt wird hierfür ein dem oberen Verstärkungsstrukturmodell 5 entsprechendes Faserbündel positioniert oder eine dem oberen Verstärkungsstrukturmodell 5 entsprechenden Form mittels des 3D-Druckers aus einem kurzfaserverstärkten Komposit-Material an der geplanten Position erzeugt.

Ein oberer Teil 10.3 des Zahnersatzteils 10 gemäß dem oberen Teilvolumen 8 des Zahnersatzteilmodells 2 mittels des 3D-Druckers 9 aus dem ersten Komposit-Material auf den mittleren Teil 10.2 des Zahnersatzteils 10 mit der angeordneten oberen Verstärkungsstruktur 12 gesetzt.

Es versteht sich, dass zur Herstellung eines Zahnersatzteils 10 mit nur einem ersten verstärkenden Faserbündel 4, das Zahnersatzteilmodell 2 nur entlang des ersten Faserbündels 4 in ein unteres Teilvolumen 6 und ein oberes Teilvolumen 8 aufgeteilt wird, so das auch das obere Teilvolumen 8 an das erste Faserbündel 4 angrenzt und der obere Teil 10.3 des Zahnersatzteils 10 gemäß dem oberen Teilvolumen 8 direkt auf das untere Teil 10.2 mit der angeordneten ersten Verstärkungsstruktur 11 gesetzt wird.

Werden mehr als zwei Verstärkungsstrukturmodelle 4, 5 zur Verstärkung in dem Zahnersatzteilmodell 2 angeordnet, so wird das Zahnersatzteilmodell in entsprechend mehr als drei Teilvolumen aufgeteilt, wobei mit jedem weiteren Faserbündel ein weiteres mittleres Teilvolumen und ein entsprechender Herstellungsschritt mit Drucken und Anordnen oder Erzeugen der weiteren Verstärkungsstruktur hinzukommt.

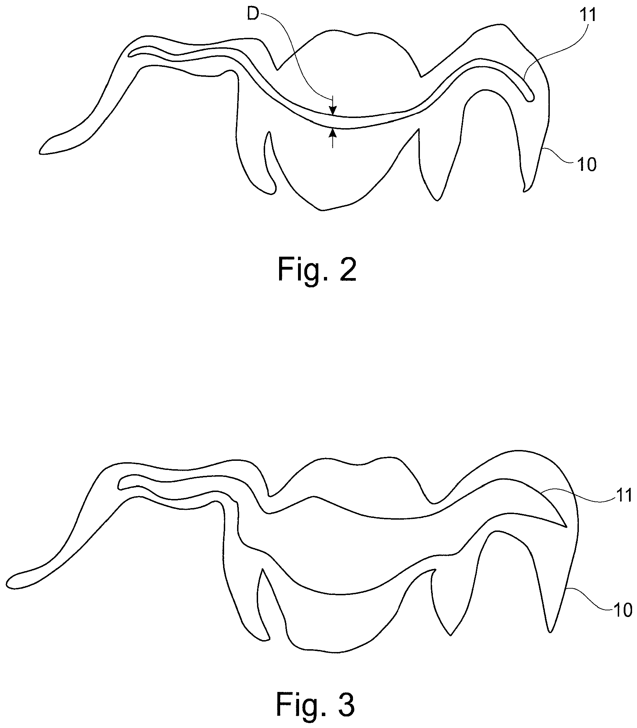

In Fig. 2 ist eine erste Ausführungsform eines durch das erfindungsgemäße Verfahren hergestellten Zahnersatzteils 10 schematisch dargestellt. Das Zahnersatzteil 10 ist als dreigliedrige Brücke mit einer einzigen Glasfasereinlage als Verstärkungsstruktur 11 ausgebildet. Die Brücke 10 ist mittels eines 3D-Druckers aus einem Komposit-Material gedruckt. Das Glasfaserbündel 11 einer Dicke D erstreckt sich im inneren der Brücke parallel zu einer gesamten Oberseite der Brücke 10. Gemäß einer Weiterbildung wird durch einen festlegbaren Mindestabstand des Glasfaserbündels 11 zu jeder Außenfläche des Zahnersatzteils 10, also beispielsweise zu der Oberseite der Brücke 10, sichergestellt, dass das Faserbündel immer von einer ausreichend dicken Schicht Komposit-Materials bedeckt ist.

In Fig. 3 ist eine zweite Ausführungsform eines durch das erfindungsgemäße Verfahren hergestellten Zahnersatzteils 10 schematisch dargestellt. Das Zahnersatzteil 10 ist als dreigliedrige Brücke mit einer einzigen Verstärkungsstruktur 11 aus einem kurzfaserverstärkten Komposit-Material ausgebildet.

## Patentansprüche

1. Verfahren zur Herstellung eines Zahnersatzteils (10) mittels eines 3D-Druckers, wobei
- ein dreidimensionales digitales Zahnersatzteilmodell (2) unter Berücksichtigung eines digitalen Gebissmodells (3) computergestützt erzeugt wird,
- das digitale Gebissmodell (3) einen zu versorgenden bezahnten und/oder unbezahnten Bereich eines Unterkiefers und/oder Oberkiefers umfasst,
- unter Berücksichtigung des Gebissmodells (3) computergestützt eine Belastungsuntersuchung des Zahnersatzteilmodells (2) durchgeführt wird,
- unter Berücksichtigung eines Ergebnisses der Belastungsuntersuchung ein dreidimensionales digitales erstes Verstärkungsstrukturmodell (4) mit einer ersten dreidimensionalen Form und einer ersten Position computergestützt innerhalb des Zahnersatzteilmodells (2) angeordnet und das Zahnersatzteilmodell (2) um ein dem angeordneten erste Verstärkungsstrukturmodell (4) entsprechendes Volumen reduziert wird,
- das reduzierte Zahnersatzteilmodell (2) in ein unteres Teilvolumen (6) und ein oberes Teilvolumen (8) aufgeteilt wird,
- das untere Teilvolumen (6) und das obere Teilvolumen (8) jeweils an das angeordnete erste Verstärkungsstrukturmodell (4) angrenzen,
- ein unterer Teil (10.1) des Zahnersatzteils (10) gemäß dem unteren Teilvolumen (6) des Zahnersatzteilmodells (10) mittels des 3D-Druckers (9) aus einem ersten Komposit-Material erzeugt wird,
- eine dem ersten Verstärkungsstrukturmodell (4) entsprechende erste Verstärkungsstruktur (11) auf dem unteren Teil (10.1) des Zahnersatzteils (10) entsprechend der ersten Position angeordnet wird,
- ein oberer Teil (10.3) des Zahnersatzteils (10) gemäß dem oberen Teilvolumen (8) des Zahnersatzteilmodells (2) mittels des 3D-Druckers (9) aus dem ersten Komposit-Material auf den unteren Teil (10.1) des Zahnersatzteils (10) mit der angeordneten ersten Verstärkungsstruktur (11) gesetzt wird, um das Zahnersatzteil (10) fertig zu stellen.

2. Verfahren nach Anspruch 1, **dadurch gekennzeichnet, dass**
- mindestens ein weiteres Verstärkungsstrukturmodell (5) mit einer weiteren Form und einer weiteren Position oberhalb und/oder neben dem ersten Verstärkungsstrukturmodell (4) innerhalb des Zahnersatzteilmodells (2) computergestützt angeordnet wird,
- das Zahnersatzteilmodell (2) um die dem angeordneten erste Verstärkungsstrukturmodell (4) und dem mindestens einen weiteren Verstärkungsstrukturmodell (5) entsprechenden Volumina reduziert wird,
- das reduzierte Zahnersatzteilmodell (2) in ein unteres Teilvolumen (6) und ein oberes Teilvolumen (8) und für jedes oberhalb der ersten Verstärkungsstruktur positionierte weitere Verstärkungsstrukturmodell in ein weiteres mittleres Teilvolumen (7) aufgeteilt wird,
- schrittweise jeweils ein Teil (10.1, 10.2, 10.3) des Zahnersatzteils (10) gemäß einem der Teilvolumina (6, 7, 8) mittels eines 3D-Druckers (9) aus dem ersten Komposit-Material erzeugt und eine einem der Verstärkungsstrukturmodelle (4, 5) entsprechende Verstärkungsstruktur (11, 12) an der jeweiligen Position angeordnet wird.

3. Verfahren nach Anspruch 1 oder 2, **dadurch gekennzeichnet**, die Belastungsuntersuchung mittels der Finite-Elemente-Methode durchgeführt wird.

4. Verfahren nach einem der Ansprüche 1 bis 3, **dadurch gekennzeichnet, dass** für die Belastungsuntersuchung zu dem zu versorgenden Kieferbereich benachbarte und/oder gegenüberliegende Zähne und/oder eine Kaubewegung berücksichtigt werden.

5. Verfahren nach einem der Ansprüche 1 bis 4, **dadurch gekennzeichnet, dass** das untere Teilvolumen (6) zumindest eine Vertiefung zur Aufnahme des ersten Verstärkungsstrukturmodells (4) und/oder jedes mittlere Teilvolumen (7) zumindest eine Vertiefung zur Aufnahme eines weiteren Verstärkungsstrukturmodells (5) aufweisen.

6. Verfahren nach einem der Ansprüche 1 bis 5, **dadurch gekennzeichnet, dass** das Gebissmodell (3) anhand von mehreren zweidimensionalen optischen Aufnahmen berechnet wird.

7. Verfahren nach einem der Ansprüche 1 bis 6, **dadurch gekennzeichnet, dass** das erste Komposit-Material biokompatibel ist.

8. Verfahren nach einem der Ansprüche 1 bis 7, **dadurch gekennzeichnet, dass** das erste Komposit-Material aus einer organischen Kunststoffmatrix und anorganischen Füllkörpern besteht, wobei ein Massenanteil der organischen Kunststoffmatrix höchstens 40% und ein Massenanteil der anorganischen Füllkörper mindestens 60% beträgt.

9. Verfahren nach einem der Ansprüche 1 bis 8, **dadurch gekennzeichnet, dass** das Zahnersatzteil mittels eines Photopolymerisationsverfahrens gedruckt wird und das erste Komposit-Material Initiatoren und Stabilisatoren für das Photopolymerisationsverfahren enthält.

10. Verfahren nach einem der Ansprüche 1 bis 9, **dadurch gekennzeichnet, dass** das mindestens ein Verstärkungsstrukturmodell (4, 5) als ein dreidimensionales digitales Faserbündelmodell ausgebildet ist, die Form des mindestens einen Verstärkungsstrukturmodells (4, 5) eine Dicke und eine Länge aufweist und ein dem mindestens einen Faserbündelmodell entsprechendes Faserbündel als Verstärkungsstruktur (11, 12) auf dem unteren Teil (10.1) des Zahnersatzteils (10) oder einem mittleren Teil (10.2) des Zahnersatzteils (10) angeordnet wird.

11. Verfahren nach Anspruch 10, **dadurch gekennzeichnet, dass** mindestens ein Faserbündel Glasfasern oder Kohlefasern oder Keramikfasern oder Fasern aus Aluminiumoxid oder eine Kombination unterschiedlicher Fasern umfasst.

12. Verfahren nach Anspruch 10 oder 11, **dadurch gekennzeichnet, dass** das mindestens eine Faserbündel mehrere in einer Matrix aus biokompatiblem Material eingebettete Fasern umfasst.

13. Verfahren nach Anspruch 12, **dadurch gekennzeichnet, dass** die Matrix aus einem organischen Harz besteht und/oder Initiatoren und Stabilisatoren für ein Photopolymerisationsverfahren beinhaltet.

14. Verfahren nach einem der Ansprüche 10 bis 13, **dadurch gekennzeichnet, dass** die Dicke des mindestens einen Faserbündelmodells aus einer vorgegebenen Menge von Dicken ausgewählt wird.

15. Verfahren nach einem der Ansprüche 10 bis 14, **dadurch gekennzeichnet, dass** das reduzierte Zahnersatzteilmodell (2) so in Teilvolumina (6, 7, 8) aufgeteilt wird, dass alle Teilvolumina (6, 7, 8) in an eine der Verstärkungsstrukturmodelle (4, 5) angrenzenden Bereichen keine Hinterschnitte aufweisen.

16. Verfahren nach einem der Ansprüche 1 bis 9, **dadurch gekennzeichnet, dass** die Verstärkungsstruktur (11, 12) mittels des 3D-Druckers aus einem kurzfaserverstärkten Komposit-Material erzeugt wird.

## Claims

1. A method for producing a dental prosthesis part (10) by means of a 3D printer, wherein
- a three-dimensional digital dental prosthesis part model (2) is generated with the aid of a computer, taking into account a digital denture model (3),
- the digital denture model (3) comprises a toothed and/or untoothed region of a lower and/or upper jaw to be treated,
- a load test of the dental prosthesis part model (2) is carried out with the aid of a computer, taking into account the denture model (3),
- a three-dimensional digital first reinforcement structure model (4) having a first three-dimensional shape and a first position is arranged with the aid of a computer within the dental prosthesis part model (2), taking into account a result of the load test, and the dental prosthesis model (2) is reduced by a volume corresponding to the arranged first reinforcement structure model (4),
- the reduced dental prosthesis part model (2) is divided into a lower partial volume (6) and an upper partial volume (8),
- the lower partial volume (6) and the upper partial volume (8) each adjoin the arranged first reinforcement structure model (4),
- a lower part (10.1) of the dental prosthesis part (10) according to the lower partial volume (6) of the dental prosthesis part model (10) is produced by means of the 3D printer (9) from a first composite material,
- a first reinforcement structure (11) corresponding to the first reinforcement structure model (4) is arranged on the lower part (10.1) of the dental prosthesis part (10) corresponding to the first position,
- an upper part (10.3) of the dental prosthesis part (10) according to the upper partial volume (8) of the dental prosthesis part model (2) of the first composite material is placed by means of the 3D printer (9) on the lower part (10.1) of the dental prosthesis part (10) with the arranged first reinforcement structure (11) in order to complete the dental prosthesis part (10).

2. The method according to claim 1, **characterised in that**
- at least one further reinforcement structure model (5) with a further shape and a further position above and/or next to the first reinforcement structure model (4) is arranged within the dental prosthesis part model (2) with the aid of a computer,
- the dental prosthesis part model (2) is reduced by the volumes corresponding to the arranged first reinforcement structure model (4) and the at least one further reinforcement structure model (5),
- the reduced dental prosthesis part model (2) is divided into a lower partial volume (6) and an upper partial volume (8) and into a further central partial volume (7), for each additional reinforcement structure model positioned above the first reinforcement structure,
- one part (10.1, 10.2, 10.3) of the dental prosthesis part (10) is gradually generated from the first composite material according to one of the partial volumes (6, 7, 8) by means of a 3D printer (9) and a reinforcement structure (11, 12) corresponding to one of the reinforcement structure models (4, 5) is arranged at the respective position.

3. The method according to claim 1 or 2, **characterised in that** the stress test is carried out by means of the finite element method.

4. The method according to one of claims 1 to 3, **characterised in that** teeth that are adjacent and/or opposite to the jaw region to be treated and/or a chewing movement are taken into account for the stress test.

5. The method according to one of claims 1 to 4, **characterised in that** the lower partial volume (6) has at least one recess for receiving the first reinforcement structure model (4) and/or each central partial volume (7) has at least one recess for receiving a further reinforcement structure model (5).

6. The method according to one of claims 1 to 5, **characterised in that** the denture model (3) is calculated on the basis of a plurality of two-dimensional optical recordings.

7. The method according to one of claims 1 to 6, **characterised in that** the first composite material is biocompatible.

8. The method according to one of claims 1 to 7, **characterised in that** the first composite material consists of an organic plastic matrix and inorganic fillers, wherein a mass fraction of the organic plastic matrix is at most 40% and a mass fraction of the inorganic filler is at least 60%.

9. The method according to one of claims 1 to 8, **characterised in that** the dental prosthesis part is printed by means of a photopolymerisation process and the first composite material contains initiators and stabilisers for the photopolymerisation process.

10. The method according to one of claims 1 to 9, **characterised in that** the at least one reinforcement structure model (4, 5) is designed as a three-dimensional digital fibre bundle model, the shape of the at least one reinforcement structure model (4, 5) has a thickness and a length and a fibre bundle corresponding to the at least one fibre bundle model is arranged as a reinforcing structure (11, 12) on the lower part (10.1) of the dental prosthesis part (10) or on a central part (10.2) of the dental prosthesis part (10).

11. The method according to claim 10, **characterised in that** at least one fibre bundle comprises glass fibres or carbon fibres or ceramic fibres or fibres made of aluminium oxide or a combination of different fibres.

12. The method according to claim 10 or 11, **characterised in that** the at least one fibre bundle comprises a plurality of fibres embedded in a matrix made of biocompatible material.

13. The method according to claim 12, **characterised in that** the matrix consists of an organic resin and/or contains initiators and stabilisers for a photopolymerisation process.

14. The method according to one of claims 10 to 13, **characterised in that** the thickness of the at least one fibre bundle model is selected from a predetermined set of thicknesses.

15. The method according to one of claims 10 to 14, **characterised in that** the reduced dental prosthesis part model (2) is divided into partial volumes (6, 7, 8) in such a way that all partial volumes (6, 7, 8) in regions adjoining one of the reinforcement structure models (4, 5) have no undercuts.

16. The method according to one of claims 1 to 9, **characterised in that** the reinforcement structure (11, 12) is produced by means of the 3D printer from a short fibre-reinforced composite material.

## Revendications

1. Procédé de fabrication d'une prothèse dentaire (10) au moyen d'une imprimante 3D, dans lequel
- un modèle de prothèse dentaire (2) numérique tridimensionnel est généré de manière assistée par ordinateur en fonction d'un modèle de dentition (3) numérique,
- le modèle de dentition (3) numérique comprend une zone dentée et/ou édentée d'une mâchoire inférieure et/ou d'une mâchoire supérieure à traiter,
- en fonction du modèle de dentition (3), un examen des charges assisté par ordinateur du modèle de prothèse dentaire (2) est effectué,
- en fonction d'un résultat de l'examen des charges, un premier modèle de structure de renfort (4) numérique tridimensionnel, présentant une première forme tridimensionnelle et une première position, est agencé de manière assistée par ordinateur dans le modèle de prothèse dentaire (2) et le modèle de prothèse dentaire (2) est réduit d'un volume correspondant au premier modèle de structure de renfort (4) agencé,
- le modèle de prothèse dentaire (2) réduit est divisé en un volume partiel inférieur (6) et un volume partiel supérieur (8),
- le volume partiel inférieur (6) et le volume partiel supérieur (8) sont respectivement contigus au premier modèle de structure de renfort (4) agencé,
- une partie inférieure (10.1) de la prothèse dentaire (10) selon le volume partiel inférieur (6) du modèle de prothèse dentaire (10) est réalisée au moyen de l'imprimante 3D (9) à partir d'une première matière composite,
- une première structure de renfort (11) correspondant au premier modèle de structure de renfort (4) est disposée sur la partie inférieure (10.1) de la prothèse dentaire (10) correspondant à la première position,
- une partie supérieure (10.3) de la prothèse dentaire (10) selon le volume partiel supérieur (8) du modèle de prothèse dentaire (2) réalisée au moyen de l'imprimante 3D (9) à partir de la première matière composite est agencée sur la partie inférieure (10.1) de la prothèse dentaire (10) avec la première structure de renfort (11) pour compléter la prothèse dentaire (10).

2. Procédé selon la revendication 1, **caractérisé en ce que**
- au moins un autre modèle de structure de renfort (5), présentant une autre forme et une autre position au-dessus et/ou à côté du premier modèle de structure de renfort (4), est agencé de manière assistée par ordinateur à l'intérieur du modèle de prothèse dentaire (2),
- le modèle de prothèse dentaire (2) est réduit des volumes correspondant au premier modèle de structure de renfort (4) agencé et à au moins un autre modèle de structure de renfort (5),
- le modèle de prothèse dentaire (2) réduit est divisé en un volume partiel inférieur (6) et un volume partiel supérieur (8) et pour chaque autre modèle de structure de renfort positionné au-dessus de la première structure de renfort en un autre volume partiel médian (7),
- une partie (10.1, 10.2, 10.3) de la prothèse dentaire (10) est respectivement générée de manière progressive selon l'un des volumes partiels (6, 7, 8) au moyen d'une imprimante 3D (9) à partir de la première matière composite et une structure de renfort (11, 12) correspondant à l'un des modèles de structure de renfort (4, 5) est agencée à la position respective.

3. Procédé selon la revendication 1 ou 2, **caractérisé en ce que** l'examen des charges est réalisé selon le procédé d'éléments finis.

4. Procédé selon l'une quelconque des revendications 1 à 3, **caractérisé en ce que** des dents adjacentes et/ou opposées à la zone de mâchoire à traiter et/ou un mouvement de mastication sont pris en compte lors de l'examen des charges.

5. Procédé selon l'une quelconque des revendications 1 à 4, **caractérisé en ce que** le volume partiel inférieur (6) comporte au moins un évidement destiné à la réception du premier modèle de structure de renfort (4) et/ou chaque volume partiel médian (7) comporte au moins un évidement destiné à la réception d'un autre modèle de structure de renfort (5).

6. Procédé selon l'une quelconque des revendications 1 à 5, **caractérisé en ce que** le modèle de dentition (3) est calculé à partir de plusieurs images optiques bidimensionnelles.

7. Procédé selon l'une quelconque des revendications 1 à 6, **caractérisé en ce que** la première matière composite est biocompatible.

8. Procédé selon l'une quelconque des revendications 1 à 7, **caractérisé en ce que** la première matière composite est constituée d'une matrice en matière plastique organique et de charges inorganiques, dans lequel une fraction massique de la matrice en matière plastique organique est d'au plus 40 % et une fraction massique de la charge inorganique est d'au moins 60 %.

9. Procédé selon l'une quelconque des revendications 1 à 8, **caractérisé en ce que** la prothèse dentaire est imprimée selon un procédé de photopolymérisation et la première matière composite contient des initiateurs et des stabilisants pour le procédé de photopolymérisation.

10. Procédé selon l'une quelconque des revendications 1 à 9, **caractérisé en ce que** l'au moins un modèle de structure de renfort (4, 5) est conçu sous la forme d'un modèle numérique tridimensionnel de faisceau de fibres, la forme de l'au moins un modèle de structure de renfort (4, 5) présente une épaisseur et une longueur et au moins un faisceau de fibres correspondant à un modèle de faisceau de fibres est agencé en tant que structure de renfort (11, 12) sur la partie inférieure (10.1) de la prothèse dentaire (10) ou sur une partie médiane (10.2) de la prothèse dentaire (10).

11. Procédé selon la revendication 10, **caractérisé en ce que** l'au moins un faisceau de fibres comprend des fibres de verre ou des fibres de carbone ou des fibres de céramique ou des fibres d'oxyde d'aluminium ou une combinaison de différentes fibres.

12. Procédé selon la revendication 10 ou 11, **caractérisé en ce que** l'au moins un faisceau de fibres comprend une pluralité de fibres noyées dans une matrice en matière biocompatible.

13. Procédé selon la revendication 12, **caractérisé en ce que** la matrice est constituée d'une résine organique et/ou contient des initiateurs et des stabilisants pour un procédé de photopolymérisation.

14. Procédé selon l'une quelconque des revendications 10 à 13, **caractérisé en ce que** l'épaisseur de l'au moins un modèle de faisceau de fibres est choisie parmi une plage d'épaisseurs prédéterminée.

15. Procédé selon l'une quelconque des revendications 10 à 14, **caractérisé en ce que** le modèle de prothèse dentaire (2) réduit est divisé en volumes partiels (6, 7, 8) de telle sorte que tous les volumes partiels (6, 7, 8) ne comportent aucune contre-dépouille dans les zones adjacentes à l'un des modèles de structure de renfort (4, 5).

16. Procédé selon l'une quelconque des revendications 1 à 9, **caractérisé en ce que** la structure de renfort (11, 12) est réalisée au moyen de l'imprimante 3D à partir d'une matière composite renforcée par des fibres courtes.
